# EUROPEAN PATENT SPECIFICATION

(11) **EP 1 631 533 B1**
(45) Date of publication and mention of the grant of the patent: **11.03.2009**
(21) Application number: 03816679.9
(22) Date of filing: 22.04.2003
(51) Int. Cl.: C07B 31/00, C07D 207/34, C07D 209/24, C07D 213/55, C07D 215/14, C07D 239/42

(54) **NOVEL PROCESS FOR STEREOSELECTIVE REDUCTION OF BETA-KETOESTERS**
NEUES VERFAHREN ZUR STEREOSELEKTIVEN REDUKTION VON BETA-KETOESTERN
NOUVEAU PROCEDE POUR UNE REDUCTION STEREOSELECTIVE DE BETA-SETOESTERS

(43) Date of publication of application: 08.03.2006
(73) Proprietor: Biocon Limited, Hebbagodi Bangalore 561 229 Karnataka (IN)
(72) Inventor: PUTHIAPARAMPIL, Tom, Thomas, c/o Biocon India Ltd., Bangalore 561 229, Karnataka (IN); POORNAPRAJNA, Acharya, c/o Biocon India LImited, Bangalore 561 229, Karnataka (IN); ASWATHANARAYANAPPA, Chandrashekar, Bangalore 561 229, Karnataka (IN); SRIDHARAN, Madhavan, c/o Biocon India Limited, Karnataka (IN); GANESH, Sambasivam, c/o Biocon India Limited, Bangalore 561 229, Karnatka (IN)
(74) Representative: Freyria Fava, Cristina
(86) International application number: PCT/IN2003/000166
(87) International publication number: WO 2004/094343

(56) References cited:
- EP-A1- 0 424 929
- US-A- 4 645 854
- US-A1- 5 200 530
- US-A1- 5 347 039
- US-B1- 6 433 213
- BONADIES F. ET AL.: 'Studies an asymetric synthesis of beta-hydroxy-delta-lactone inhibitors of HMGCoA reductase 1: A new preparation of the lactone moiety of compactin' TETRAHEDRON LETTERS vol. 28, no. 6, 1987, pages 703 - 706, XP003010365
- BOUTIN R.H. ET AL.: 'Alpha amino acid derivatives as chiral educts for asymetric products. Synthesis of Sphingosine from alpha-amino-alpha,beta-yones' J. ORG. CHEM. vol. 51, no. 26, 1986, pages 5320 - 5327, XP002272598
- HAMADA YASUMASA ET AL.: 'New methods and reagents in organic synthesis. 29. a practical method for the preparation of optically active N-protected alpha-amino aldehydes and peptide aldehydes' CHEM. PHARM. BULL. vol. 30, no. 5, 1982, pages 1921 - 1924, XP008079442
- LUCHE JEAN-LOUIS: 'Lanthanides in organic chemistry,1. Selective 1,2 reductions of conjugated ketones' J. AM. CHEM. SOC. vol. 100, no. 7, 1978, pages 2226 - 2227, XP003010366
- RUCKER G. ET AL.: 'Stereoselective reduction of cyclic 2,3-epoxyketones to trans-2,3-epoxyalcohols' SYNTHETIC COMMUNICATIONS vol. 10, no. 8, 1980, pages 623 - 626, XP008079453

## Description

### TECHNICAL FIELD

The present invention relates to a novel stereoselective process for preparing optically active dihydroxy ester derivatives of formula I which are useful intermediates for the synthesis of HMG-CoA enzyme inhibitors like atorvastatin, cerivastatin, rosuvastatin, itavastatin, fluvastatin.

### BACKGROUND

Esters derivatives of the formula I 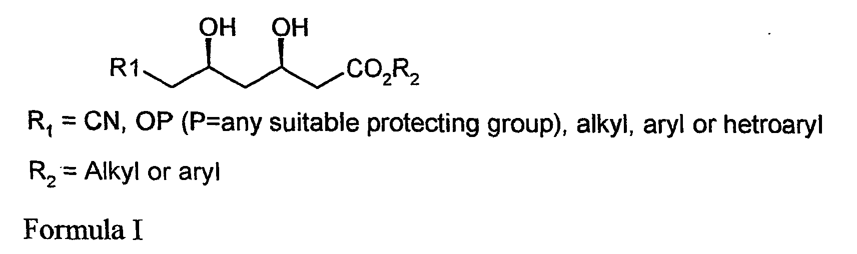 are valuable chiral synthons for synthesizing compounds which are known anti-hyptercholesterolemic agents having an inhibitory effect on HMG-CoA reductase (Ref. US 5003080, US 5169857, WO 01 85702, US 5354772, EP 0304063)

The most common approach for achieving stereoselective synthesis of compounds of formula I is the reduction of formula II 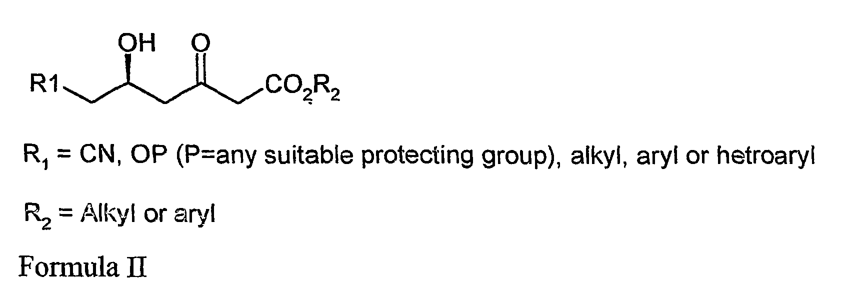 using special borane reagents (ref US 5273995, US 5470981, US 5489691). Reagents such as methoxydiethylborane are hazardous and expensive.

US 6001615 describe an enzymatic route of synthesis. This process, however, suffers from the fact that the process is not industrially scalable and also involves large volumes.

US 5399722 describe a process starting from commercially available ethyl-ω-chloroacetoacetate or its benzyloxy derivative. The disadvantages of this process is that a stereoselective reduction using a ruthenium-BINAP catalyst in employed and the desired compound of formula I is obtained in six steps.

US 5481009 describe a process starting from 4-phenyl-3-butenoic acid about 5 steps. The process uses hazardous steps (ozonolysis) to obtain the desired product. Other processes for the reduction of β-ketoesters are known i.a. from US-A-5 347 039 and Bonini et al., J. Org. Chem, 1991, 56, 4050-52.

The objective of the present invention is to provide a simple, industrially scalable process for the stereoselective reduction of compounds of formula II, leading to preparation of compounds of formula I employing inexpensive and non-hazardous reagents.

The use of compounds of formula I in synthesis of statins are illustrated in Schemes 1-6.

The present invention has following advantages over known method:
1. Safe and non-hazardous
2. Cost-effective
3. Industrially scalable
4. Lesser number of steps
5. Commercially viable

### Summary of the invention

The present invention details a novel process for the preparation of compounds of formula I 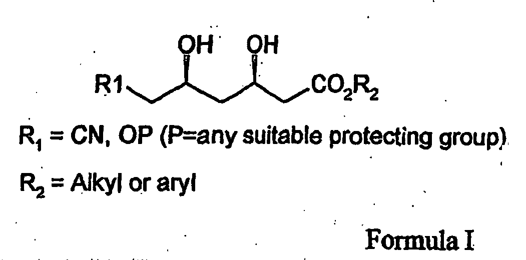 by reacting a compound of formula II 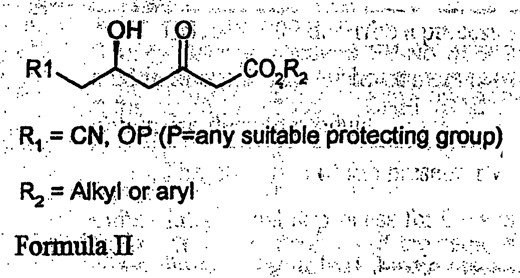
R₁= CN, OP (P=any suitable protecting group)
R₂ = Alkyl or aryl
Formula II
with sodium borohydride in presence of CeCl₃ or Ti(OiPr)₄ (Scheme I). The reagents used are non-hazardous, easily available and cheap.

### Detailed Description of the invention

The compound of formula II is an important intermediate for the preparation of many drug molecules especially, HMG Co-A reductase inhibitors. The HMG Co-A reductase inhibitors are useful as inhibitors of the enzyme 3-hydroxy-3-methylglutaryl-coenzyme A reductase (HMG CoA reductase) and are thus useful as hypolipidemic or hypocholesterolemic agents.

The process of the present invention in its first aspect is a new, improved, non-hazardous, industrially scalable, economical, and commercially feasible method for preparing intermediates used for the preparation of HMG CoA reductase inhibitors. The process of the present invention in its first aspect is outlined in Scheme 1.

The present invention details a novel process for the preparation of compounds of formula I 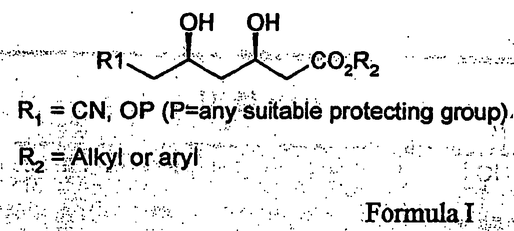 by reacting a compound of formula II 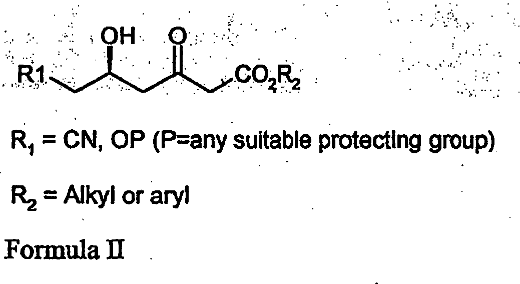 with
1) sodium borohydride in presence of anhydrous CeCl₃ or
2) hydrated CeCl₃ or
3) Ti(OiPr)₄

The reagents used are non-hazardous, easily available and economic.

Compounds of formula I are important intermediates for the preparation of HMG Co-A reductase inhibitors as shown in Scheme 2 (Atorvastatin), Scheme 3 (Cerivastatin), Scheme 4 (Itavastatin), Scheme 5 (Rosuvastatin) and Scheme 6 (Fluvastatin).

The following examples illustrate the inventors' preferred method for preparing the compounds of the invention.

### EXAMPLES

### Example 1

### Preparation of tert-butyl (3R,5R)-6-cyano-3,5-dihydroxyhexanoate:

A solution of *tert*-butyl (5*R*)-6-cyano-5-hydroxy-3-oxohexanoate (10 g, 0.044 mol) in THF (60 mL) was stirred under nitrogen and methanol (20 mL) was added. The reaction mixture was stirred for 15 min. and cooled to -50° C to -55° C. Anhydrous CeCl₃ (10.8 g, 0.044 mol) was added and stirred for 30 min., maintaining temperature between -50 to -55 ° C. Sodium borohydride (2.5 g, 0.066 mol) was added in 6 portions maintaining the temperature (-70 to -90°C) and stirred for 1 h at the same temperature. After warming the reaction mixture to RT, it was concentrated to residue under vacuum at about 45° C. Methanol (60 mL) was added and concentrated completely. The residue was cooled to RT, water (50 mL) was added and extracted with ethyl acetate (100 mL x 2). The-combined-organic layer was washed with brine solution (50 mL), concentrated to obtain title compound. Yield: 9 g.

### Example 2

### Preparation of tert-butyl (3R,5R)-6-cyano-3,5-dihydroxyhexanoate:

A solution of *tert*-butyl (5*R*)-6-cyano-5-hydroxy-3-oxohexanoate (10 g, 0.044 mol) in THF (60 mL) was stirred under nitrogen and methanol (20 mL) was added. The reaction mixture was stirred for 15 min. and cooled to -50° C to -55 ° C. CeCl₃.7H₂O (16.4 g, 0.044 mol) was added and stirred for 30 min., maintaining temperature between -50 to -55 ° C. Sodium borohydride (2.5 g, 0.066 mol) was added in 6 portions maintaining the temperature (-70 to -90°C) and stirred for 1 h at the same temperature. After warming the reaction mixture to RT, it was concentrated to residue under vacuum at about 45°C. Methanol (60 mL) was added and concentrated completely. The residue was cooled to RT, water (50 mL) was added and extracted with ethyl acetate (100 mL x 2). The combined organic layer was washed with brine solution (50 mL), concentrated to obtain title compound.
Yield: 5 g.

### Example 3

### Preparation of tert-butyl (3R,5R)-6-cyano-3,5-dihydroxyhexanoate:

A solution of *tert*-butyl (5R)-6-cyano-5-hydroxy-3-oxohexanoate (10 g, 0.044 mol) in THF (60 mL) was stirred under nitrogen and methanol (20 mL) was added. The reaction mixture was stirred for 15 min. Ti(IV)isopropoxide (12.5 g, 0.044 mol) was added and stirred for 30 min. at room temperature. After cooling the reaction mixture to -50° C to -55° C, sodium borohydride (1.67 g, 0.044 mol) was added in 4 portions maintaining the temperature (-50° C-to—55° C) and stirred for 1 h at the same temperature. After warming the reaction mixture to RT, it was concentrated to residue under vacuum at about 45° C. Methanol (60 mL) was added and concentrated completely. The residue was cooled to RT, water (50 mL) was added and extracted with ethyl acetate (100 mL x 2). The combined organic layer was washed with saturated ammonium chloride solution (2 x 50 mL), water (50 mL) and brine solution (50 mL), concentrated to obtain title compound. Yield: 7.5 g.

### Example 4

### Preparation of (3R,5S)-6-(tert-butyl-diphenyl-silanyloxy)-3,5-dihydroxyhexanoic acid tert-butyl ester:

A solution of (5S)-6-(tert-butyl-diphenyl-silanyloxy)-5-hydroxy-3-oxo-hexanoic acid tert-butyl ester (20 g, 0.044 mol) in THF (60 mL) was stirred under nitrogen and methanol (20 mL) was added. The reaction mixture was stirred for 15 min. and cooled to -50° C to -55° C. Anhydrous CeCl₃ (10.8 g, 0.044 mol) was added and stirred for 30 min., maintaining temperature between -50 to -55° C. Sodium borohydride (2.5 g, 0.066 mol) was added in 6 portions maintaining the temperature (-70 to -90° C) and stirred for 6 h at the same temperature. After warming the reaction mixture to RT, it was concentrated to residue under vacuum at about 45° C. Methanol (60 mL) was added and concentrated completely. The residue was cooled to RT, water (50 mL) was added and extracted with ethyl acetate (100 mL x 2). The combined organic layer was washed with brine solution (50 mL), concentrated to obtain title compound.
Yield: 17 g.

### Example 5

### Preparation of (3R,5S)-3,5-dihydroxy-6-tritrloxy- hexanoic acid tert-butyl ester:

A solution of (5S)-5-dihydroxy-3 -oxo-6-trityloxy- hexanoic acid tert-butyl ester (20 g, 0.044 mol) in THF (75 mL) was stirred under nitrogen and methanol (20 mL) was added. Ti(IV)isopropoxide (12.5 g, 0.044 mol) was added and stirred for 30 min. at room temperature. After cooling the reaction mixture to -50°C to-55° C, sodium borohydride (1.67 g, 0.044 mol) was added in 4 portions maintaining the temperature (-50°C to-55°C) and stirred for 5h at the same temperature. After warming the reaction mixture to RT, it was concentrated to residue under vacuum at about 45°C. Methanol (60 mL) was added and concentrated completely. The residue was cooled to RT, water (50 mL) was added and extracted with ethyl acetate (100 mL x 2). The combined organic layer was washed with saturated ammonium chloride solution (2 x 50 mL), water (50mL) and brine solution (50 mL), concentrated to obtain title compound. Yield: 14.5 g.

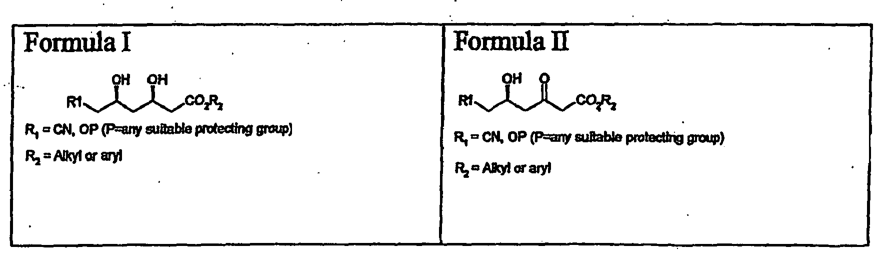

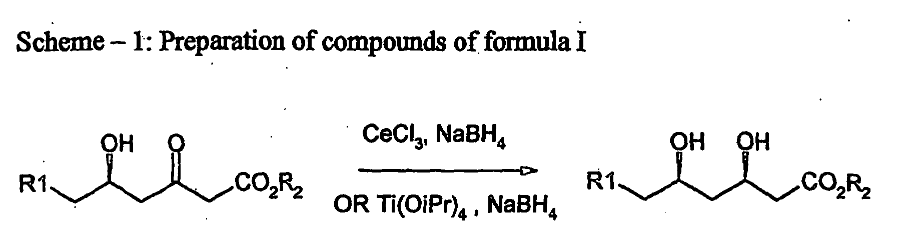

R₁ = CN, OP (P=any suitable protecting group),
R₂= Alkyl or aryl

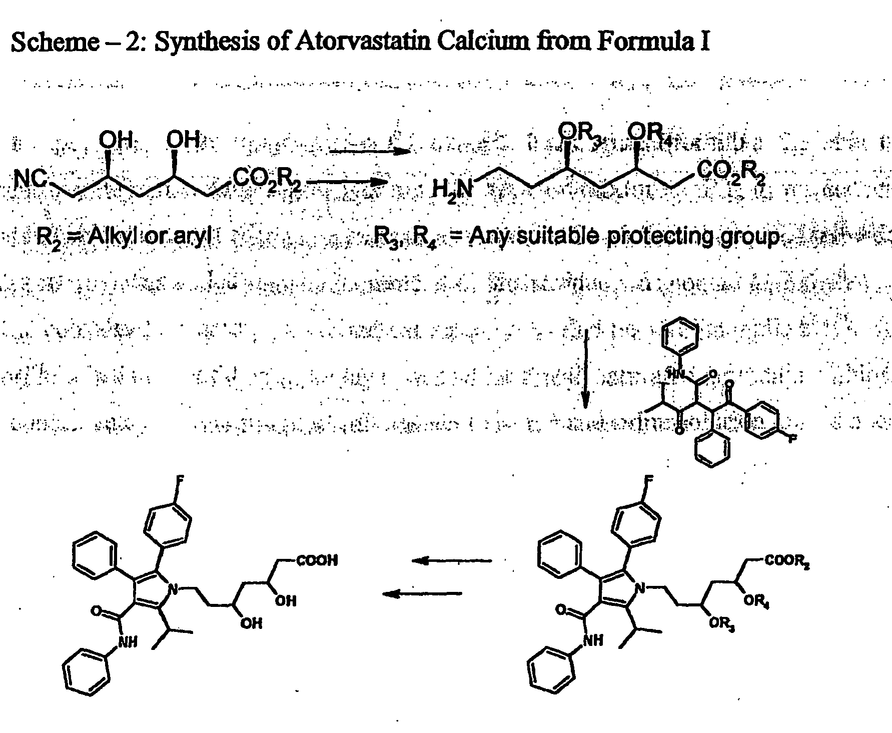

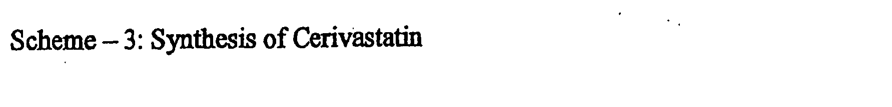

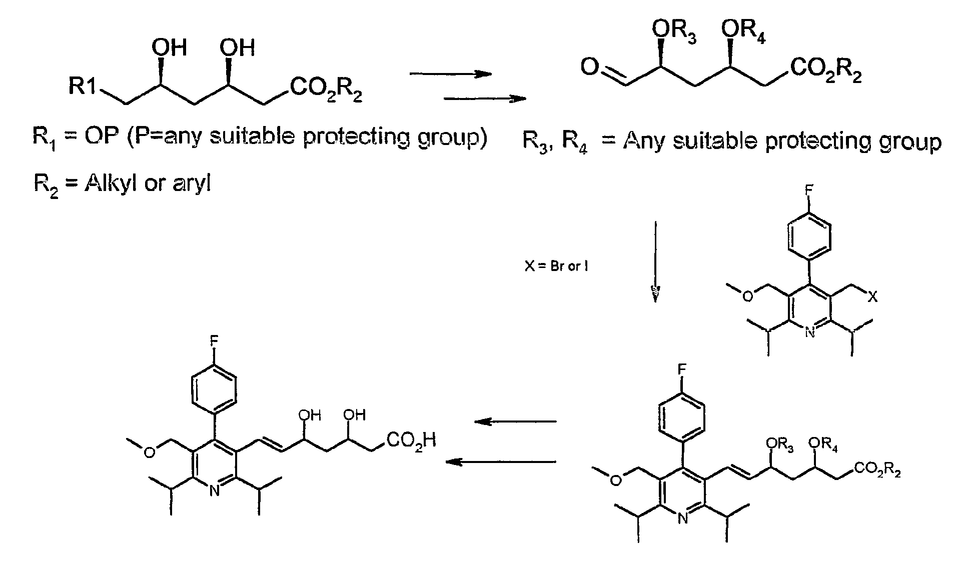

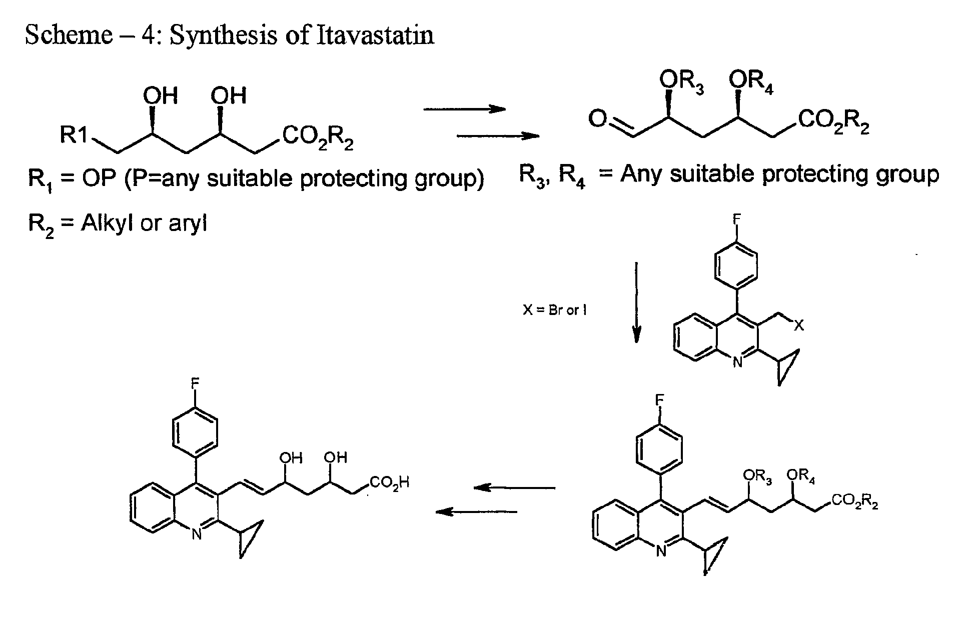

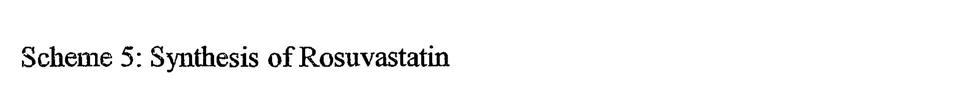

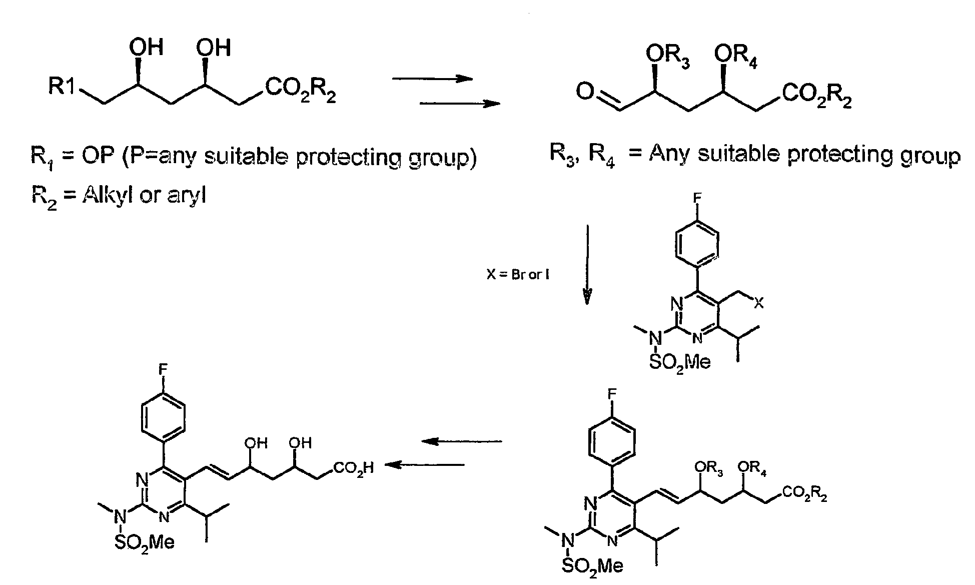

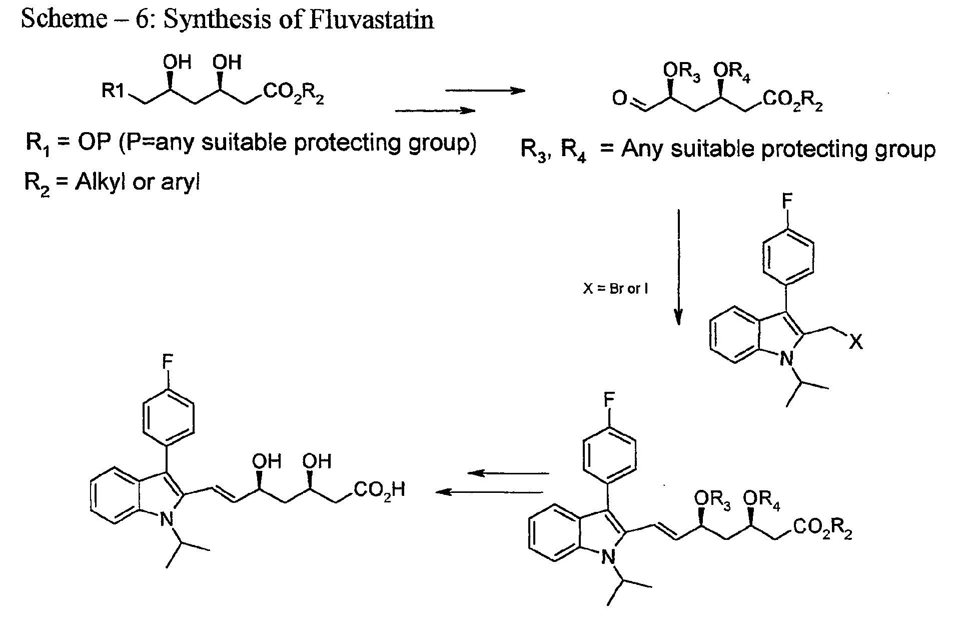

## Claims

1. A process for the preparation of compounds of formula I 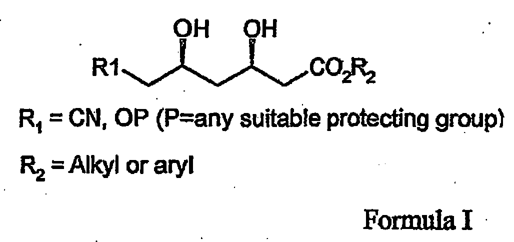 by reacting a compound of formula II 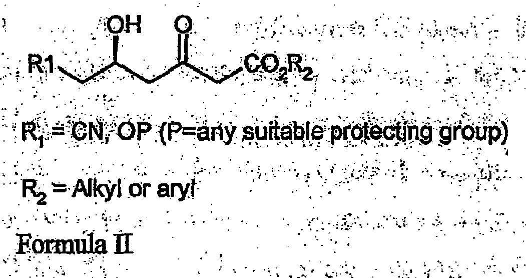 with
sodium borohydride in presence of CeCl₃ or Tᵢ(OᵢA)₄.

2. A process in claim 1, wherein the compound of formula I is further used for production of HMG CoA reductase inhibitors.

3. A process as in claim 2, wherein the HMG CoA reductase inhibitor is selected from state group comprising Atorvastatin, Rosuvastatin, Cerivastatin, Fluvastatin and Itavastatin.

## Patentansprüche

1. Verfahren zur Herstellung von Verbindungen der Formel I 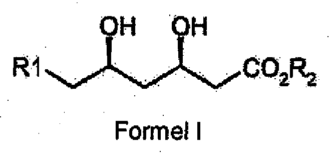
R₁ = CN, OP (P = jede geeignete Schutzgruppe)
R₂ = Alkyl oder Aryl
durch Umsetzen einer Verbindung der Formel II 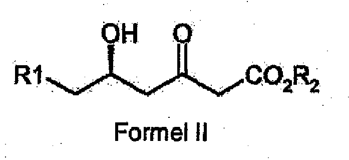
R₁ = CN, OP (P = jede geeignete Schutzgruppe)
R₂ = Alkyl oder Aryl
mit
Natriumborhydrid in Gegenwart von CeCl₃ oder Ti(OiPr)₄.

2. Verfahren nach Anspruch 1, wobei die Verbindung der Formel I außerdem für die Herstellung von HMG-CoA-Reduktase-Inhibitoren verwendet wird.

3. Verfahren nach Anspruch 2, wobei der HMG-CoA-Reduktase-Inhibitor ausgewählt ist aus der Statingruppe, umfassend Atorvastatin, Rosuvastatin, Cerivastatin, Fluvastatin und Itavastatin.

## Revendications

1. Procédé de préparation de composés de formule (I) 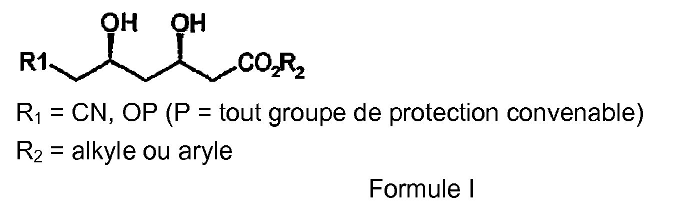 par réaction d'un composé de formule II 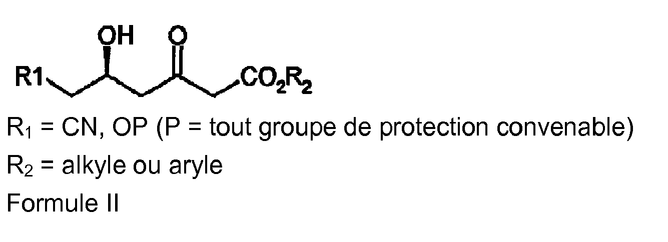 avec
un borohydrure de sodium en présence de CeCl₃ ou Ti(OiPr)₄.

2. Procédé selon la revendication 1, dans lequel le composé de formule I est, en outre, utilisé pour la production d'inhibiteurs d'HMG coenzyme A réductase.

3. Procédé selon la revendication 2, dans lequel l'inhibiteur d'HMG coenzyme A réductase est choisi dans le groupe des statines comprenant l'atorvastatine, la rosuvastatine, la cérivastatine, la fluvastatine et l'itavastatine.
